# Europäisches Patentamt
# European Patent Office
# Office européen des brevets

(19)

(11) Numéro de publication: **0 148 680**

**B1**

(12)

# FASCICULE DE BREVET EUROPÉEN

(45) Date de publication du fascicule du brevet:
**02.11.88**

(51) Int. Cl.⁴: **A 61 K 37/18**

(21) Numéro de dépôt: **84402594.0**

(22) Date de dépôt: **14.12.84**

(54) Solutions d'acides amines.

(30) Priorité: **20.12.83 FR 8320383**

(43) Date de publication de la demande:
**17.07.85 Bulletin 85/29**

(45) Mention de la délivrance du brevet:
**02.11.88 Bulletin 88/44**

(84) Etats contractants désignés:
**AT BE CH DE FR GB IT LI LU NL SE**

(56) Documents cité:
**WO-A-83/03969**
**US-A-3 950 529**

**Biol. Neonate 32, pages 73-76 (1977)**

(73) Titulaire: **SYNTHELABO, 58, rue de la Glacière,**
**F-75621 Paris Cedex 13 (FR)**

(72) Inventeur: **Rigo, Jacques, 111, rue de la Loignerie,**
**B-4930 Chaud- Fontaine (BE)**

(74) Mandataire: **Thouret- Lemaitre, Elisabeth, Service**
**Brevets - SYNTHELABO 58, rue de la Glacière,**
**F-75621 Paris Cédex 13 (FR)**

EP 0 148 680 B1

**Description**

La présente invention concerne des solutions d'acides aminés adaptées à l'alimentation des enfants de faible poids de naissance, nouveau-nés à terme et jeunes nourrissons, pouvant être utilisées par voie orale, entérale ou parentérale.

Le déséquilibre nutritionnel en période néonatale risque d'entraîner des perturbations définitives du développement psychomoteur. La vitesse de croissance du foetus in utero au cours des trois derniers mois de la gestation est très rapide entre la 28ème et la 36ème semaine de gestation, le foetus fixe environ 2 grammes de protéines par kg de poids corporel et par jour.

La croissance de l'enfant de faible poids de naissance dans les premières semaines de vie doit être semblable quantitativement à celle du foetus pendant les trois derniers mois de la gestation.

Pour obtenir un tel résultat, il est nécessaire d'alimenter les enfants de faible poids de naissance à l'aide, notamment, de solutions d'acides aminés spécialement adaptées à leurs besoins.

L'efficacité de cette alimentation spécifique sera jugée sur les dosages d'acides aminés plasmatiques, le bilan azoté et l'état protidique sanguin qui reflètent l'état nutritionnel protéique.

Il faut pour cela déterminer des valeurs de référence, c'est-à-dire une amino-acidémie, une rétention azotée et une protidémie normales pour l'enfant de faible poids de naissance. Chez le foetus in utero, les substrats nutritifs sont apportés par la circulation materno-foetale. Le sang du cordon représente la résultante des apports maternels, du métabolisme foetal et des échanges foeto-maternels au niveau du placenta. L'aminoacidémie du cordon est stable durant le dernier trimestre de la gestation et correspond à l'aminoacidémie foetale. Elle reflète la concentration d'acides aminés disponible pour la croissance tissulaire du foetus. Il paraît donc logique de prendre en considération l'aminoacidémie cordonale pour déterminer l'apport optimal en acides aminés pour les enfants de faible poids de naissance.

Ces taux plasmatiques d'acides aminés observés dans le sang de cordon sont une garantie de non toxicité pour le développement neurologique.

L'apport optimal en acides aminés pour l'enfant de faible poids de naissance sera celui qui procurera une aminoacidémie comparable à celle du sang de cordon des nouveau-nés à terme puisque cette aminoacidémie assure une croissance normale.

L'influence de différents apports nutritionnels sur le bilan azoté, la protidémie et l'aminoacidémie des enfants de faible poids de naissance a fait l'objet de nombreuses études. Ceci a permis de déterminer les apports protéiques procurant chez ces enfants de faible poids de naissance des taux plasmatiques d'acides aminés comparable à ceux du sang de cordon des nouveau-nés:

- quantitativement, l'apport doit être de 3,1 g/kg/jour;
- qualitativement, les quantités d'acides aminés essentiels et semi-essentiels doivent être telles que le rapport pondéral: $\frac{\text{acides aminés essentiels} + \text{acides aminés semi-essentiels}}{\text{acides aminés totaux}}$ soit de 55 %.

Les acides aminés essentiels pour l'enfant de faible poids de naissance sont les suivants : thréonine, leucine, isoleucine, valine, méthionine, phénylalanine, tryptophane, lysine et histidine.

Les acides aminés semi-essentiels pour l'enfant de faible poids de naissance sont la cystéine, la taurine et la tyrosine.

Les quantités optimales d'acides aminés essentiels et semi-essentiels ainsi déterminées pour ces enfants sont de:

|  | mg/kg/jour |
|---|---|
| Thréonine | 114 |
| Leucine | 307 |
| Isoleucine | 206 |
| Valine | 233 |
| Méthionine | 74 |
| Cystéine | 58 |
| Taurine | 18 |
| Phénylalanine | 129 |
| Tyrosine | 28 |
| Lysine | 338 |
| Histidine | 117 |
| Tryptophane | 61 |

Pour les nouveau-nés à terme et les jeunes nourrissons souffrant de différents troubles, les valeurs de référence, c'est-à-dire aminoacidémie, rétention azotée et protidémie, sont les valeurs obtenues pour une alimentation d'un nourrisson normal nourri au lait maternel. Les études montrent que, dans ce cas, quantitativement l'apport doit être de 2,5 g/kg/jour d'acides aminés et, qualitativement, les quantités d'acides aminés essentiels et semi-essentiels sont les mêmes que celles qui ont été données précédemment pour l'enfant de faible poids de naissance, compte tenu du fait que les immaturités enzymatiques persistent chez le nouveau-né pendant plusieurs semaines.

Diverses solutions d'acides aminés ont déjà été proposées dans la littérature pour l'alimentation orale, entérale et/ou parentérale.

Le brevet US-3 950 529 est relatif à des mélanges d'acides aminés essentiels et non essentiels dont les quantités molaires sont précisées dans la revendication 1 mais ne contenant pas de taurine. Ces mélanges sont destinés à des patients souffrant de maladies hépatiques et ne contiennent pas de taurine.

La demande de brevet WO 83/03969 a pour objet des solutions d'amino-acides essentiels et non-essentiels destinées aux enfants et aux adultes. Les quantités d'acides aminés sont déterminées de manière à retrouver le taux post-prandial d'un enfant normal (voir page 4, 1er paragraphe). De plus les huit acides aminés essentiels pour l'adulte ainsi que les trois acides aminés supplémentaires essentiels pour l'enfant sont définis à la page 5, 2e paragraphe. Les exemples de solutions ne contiennent pas de taurine.

La demande de brevet européen 0 147 682 est, elle aussi, relative à une solution d'amino-acides ne contenant pas de taurine et destinée à des malades en conditions postopératoires.

La Demanderesse a, par ses travaux, été amenée à constater l'intérêt d'un apport de taurine dans les solutions d'acides aminés. Voir par exemple Biol. Neonate 32: 73 - 76 (1977).

Les solutions d'acides aminés de l'invention, destinées aux enfants de faible poids de naissance, nouveau-nés à terme et jeunes nourrissons et pouvant être utilisées par voie orale, entérale ou parentérale, sont caractérisées par le fait qu'elles contiennent des acides aminés essentiels (thr, leu, ileu, val, mét, phé, tyr, lys, his) et semi-essentiels (cys, tau, tyr) dans un rapport pondéral entre les acides aminés essentiels et semi-essentiels et les acides aminés totaux de 46 à 63 % et par le fait qu'elles contiennent de la taurine en une quantité de 0,5 % 0,7 % en poids par rapport au total des acides aminés.

De plus les solutions d'acides aminés de l'invention, contiennent de 20,4 à 27,7 % en poids d'acides aminés ramifiés, de 6,0 à 8,1 %en poids d'acides aminés aromatiques, de 2,0 à 2,7 %, en poids de méthionine, de 1,6 à 2,2 %, en poids de cystéine et de 1,9 à 2,6 % en poids d'ornithine; les pourcentages en poids étant exprimés par rapport au total des acides aminés.

Les solutions d'acides aminés de l'invention sont constituées de la manière suivante (les quantités exprimées sont des pourcentages en poids):

| | | | |
|---|---|---|---|
| Isoleucine | 5,7 | à | 7,7 |
| Leucine | 8,5 | à | 11,4 |
| Valine | 6,4 | à | 8,7 |
| Lysine | 9,3 | à | 12,6 |
| Méthionine | 2,0 | à | 2,7 |
| Phénylalanine | 3,6 | à | 4,8 |
| Thréonine | 3,1 | à | 4,2 |
| Tryptophane | 1,7 | à | 2,3 |
| Arginine | 7,1 | à | 9,6 |
| Histidine | 3,2 | à | 4,4 |
| Alanine | 6,8 | à | 9,2 |
| Acide aspartique | 5,1 | à | 6,9 |
| Cystéine | 1,6 | à | 2,2 |
| Acide glutamique | 8,5 | à | 11,4 |
| Glycine | 3,4 | à | 4,6 |
| Proline | 2,5 | à | 3,4 |
| Sérine | 3,4 | à | 4,6 |
| Tyrosine | 0,4 | à | 1,0 |
| Ornithine | 1,9 | à | 2,6 |
| Taurine | 0,5 | à | 0,7 |

Le rapport pondéral $\frac{\text{acides aminés essentiels + acides aminés semi-essentiels}}{\text{acides aminés totaux}}$ va de 46 à 63 %.

Les solutions de l'invention apportent de 2 à 10 g d'acides aminés par 100 ml de solution.

Les solutions d'acides aminés de l'invention peuvent être administrées soit par voie intraveineuse sous forme d'un soluté d'acides aminés cristallisés, soit par voie entérale à l'aide d'une sonde, soit par voie orale.

Les acides aminés sont utilisés sous forme L. Certains acides amiés peuvent être présents sous forme de sels: chlorhydrate, acétate ou autre. Ces antioxydants tels que vitamine C ou hydrosulfite de sodium peuvent être ajoutés à la solution.

Les solutions de l'invention peuvent être utilisées telles quelles ou après addition d'électrolytes, d'oligoéléments, de minéraux et/ou de vitamines et/ou en association avec des solutions d'ose (glucose, maltose, fructose ou sorbitol) et/ou de lipides, en fonction des besoins nutritionnels des enfants de faible poids de naissance, nouveau-nés à terme et jeunes nourrissons.

Les exemples suivants illustrent l'invention.

Exemple 1
La solution d'acides aminés a la formule suivante:

|  | mg/100 ml |
|---|---|
| Isoleucine | 335 |
| Leucine | 500 |
| Valine | 380 |
| Lysine | 550 |
| Méthionine | 120 |
| Phénylalanine | 210 |
| Thréonine | 185 |
| Tryptophane | 100 |
| Arginine | 420 |
| Histidine | 190 |
| Alanine | 400 |
| Acide aspartique | 300 |
| Cystéine (chlorhydrate) | 123 |
| Acide glutamique | 500 |
| Glycine | 200 |
| Proline | 150 |
| Sérine | 200 |
| Tyrosine | 45 |
| Ornithine | 113 |
| Taurine | 30 |
| Eau q. s. p. | 100 ml |

Cette composition apporte 5,051 g d'acides aminés pour 100 ml.

Le rapport pondéral $\frac{\text{acides aminés essentiels + acides aminés semi-essentiels}}{\text{acides aminés totaux}}$ est égal à 55 %.

### Exemple 2
A la solution de l'exemple 1, il peut être ajouté des électrolytes, des minéraux et des oligoéléments selon les proportions suivantes:

| | |
|---|---|
| Solution d'acides aminés de l'exemple 1 | 100 ml |
| Sodium | 4 ,4 mEq |
| Potassium | 4,7 mEq |
| Chlore | 4,4 mEq |
| Calcium | 75 mg |
| Phosphore | 70 mg |
| Magnésium | 7,5 mg |
| Zinc | 0,6 mg |
| Cuivre | 0,06 mg |

### Exemple 3
La solution de l'exemple 2 peut être mélangée avec une solution de glucose à 20 % dans les quantités suivantes (1 partie pour 2 parties respectivement):
Solution d'acides aminés avec électrolytes, oligoéléments et

| | |
|---|---|
| minéraux de l'exemple 2 | 50 ml |
| Solution de glucose à 20 % | 100 ml |

### Exemple 4
La solution de l'exemple 1 peut être mélangée avec une solution de glucose à 20 % dans les quantités (1 partie pour 2 parties respectivement):

| | |
|---|---|
| Solution d'acides aminés de l'exemple 1 | 50 ml |
| Solution de glucose à 20 % | 100 ml |

### Exemple 5
La solution de l'exemple 1 peut être mélangée avec une solution de glucose à 30 % et une émulsion lipidique à 20 % selon les proportions suivantes:

| Solutions d'acides aminés de l'exemple 1 | 60 ml |
|---|---|
| Solution de glucose à 30 % | 40 ml |
| Emulsion lipidique à 20 % (par exemple huile de soja émulsionnée par des phosphatides de jaune d'oeuf ou de soja) | 10 ml |

Les solutions de l'invention sont administrées par voie orale, parentérale ou entérale de manière à apporter 3,1 g/kg/jour d'acides aminés aux enfants de faible poids de naissance ou 2,5 g/kg/jour d'acides aminés aux nouveau-nés à terme et jeunes nourrissons.

**Revendications** pour les états contractants: BE, CH, DE, FR,GB, ST, LI, LU, NL, SE.

1. Solutions d'acides aminés pour enfants de faible poids de naissance, nouveau-nés à terme et jeunes nourrissons, pouvant être utilisées par voie orale, entérale ou parentérale, solutions caractérisées par le fait qu'elles contiennent des acides aminés essentiels (thr, leu, ileu, val, mét, phé, tyr, lys, his) et semi-essentiels (cys, tau, tyr) dans un rapport pondéral entre les acides aminés essentiels et semi-essentiels et les acides aminés totaux de 46 à 63 % et par le fait qu'elles contiennent de la taurine en une quantité de 0,5 % à 0,7 % en poids par rapport au total des acides aminés.

2. Solutions d'acides aminés selon la revendication 1, caractérisées par le fait qu'elles contiennent de 20,4 à 27,7 % en poids d'acides aminés ramifiés, de 6,0 à 8,1 % en poids d'acides aminés aromatiques, de 2,0 à 2,7 % en poids de méthionine, de 1,6 à 2,2 % en poids de cystéine, et de 1,9 à 2,6 % en poids d'ornithine; les pourcentages en poids étant exprimés par rapport au total des acides aminés.

3. Solutions d'acides aminés, selon la revendication 1 ou 2, caractérisées par le fait qu'elles peuvent contenir des oses (glucose, maltose, fructose, sorbitol) , des électrolytes, des minéraux, des oligoéléments, des vitamines et/ou des lipides.

4. Solutions d'acides aminés selon l'une quelconque des revendications 1 à 3, caractérisées par le fait que la concentration en acides aminés est de 2 à 10 g/100 ml de solution.

5. Solution d'acides aminés selon l'une quelconque des revendications 1 à 4, caractérisée par le fait qu'elle contient les acides aminés suivants dans les quantités suivantes exprimées en pourcentages en poids par rapport au total des acides aminés:

| L-Isoleucine | 5,7 | à | 7,7 |
|---|---|---|---|
| L-Leucine | 8,5 | à | 11,4 |
| L-Valine | 6,4 | à | 8,7 |
| L-Lysine | 9,3 | à | 12,6 |
| L-Méthionine | 2,0 | à | 2,7 |
| L-Phénylalanine | 3,6 | à | 4,8 |
| L-Thréonine | 3,1 | à | 4,2 |
| L-Tryptophane | 1,7 | à | 2,3 |
| L-Arginine | 7,1 | à | 9,6 |
| L-Histidine | 3,2 | à | 4,4 |
| L-Alanine | 6,8 | à | 9,2 |
| Acide L-aspartique | 5,1 | à | 6,9 |
| L-Cystéine | 1,6 | à | 2,2 |
| Acide L-glutamique | 8,5 | à | 11,4 |
| Glycine | 3,4 | à | 4,6 |
| L-Proline | 2,5 | à | 3,4 |
| L-Sérine | 3,4 | à | 4,6 |
| L-Tyrosine | 0,4 | à | 1,0 |
| L-Ornithine | 1,9 | à | 2,6 |
| Taurine | 0,5 | à | 0,7 |

6. Solution selon la revendication 1, 2, 3, 4 ou 5, caractérisée par le fait qu'elle a la formule suivante:

|  | mg/100 ml |
|---|---|
| L-Isoleucine | 335 |
| L-Leucine | 500 |
| L-Valine | 380 |
| L-Lysine | 550 |
| L-Méthionine | 120 |
| L-Phénylalanine | 210 |
| L-Thréonine | 185 |
| L-Tryptophane | 100 |
| L-Arginine | 420 |
| L-Histidine | 190 |
| L-Alanine | 400 |
| Acide L-aspartique | 300 |
| L-Cystéine (chlorhydrate) | 123 |
| Acide L-glutamique | 500 |
| Glycine | 200 |
| L-Proline | 150 |
| L-Sérine | 200 |
| L-Tyrosine | 45 |
| L-Ornithine | 113 |
| Taurine | 30 |
| Eau q.s.p. | 100 ml |

7. Solution selon la revendication 3, caractérisée par le fait qu'elle est constituée par le mélange suivant:

| soluton d'acides aminés de la revendication 6 | 100 ml |
|---|---|
| sodium | 4,4 mEq |
| potassium | 4,7 mEq |
| chlore | 4,4 mEq |
| calcium | 75 mg |
| phosphore | 70 mg |
| magnésium | 7,5 mg |
| zinc | 0,6 mg |
| cuivre | 0,06 mg |

8. Solution selon la revendication 3, caractérisée par le fait qu'elle est constituée par le mélange suivant:

| Solution d'acides aminés de la revendication 7 | 50 ml |
|---|---|
| Solution d'ose à 20 % | 100 ml |

9. Solution selon la revendication 3, caractérisée par le fait qu'elle est constituée par le mélange suivant:

| Solution d'acides aminés de la revendication 6 | 60 ml |
|---|---|
| Solution de glucose à 30 % | 40 ml |
| Emulsion lipidique à 20 % | 10 ml |

**Revendications** pour l'état contractant: AT.

1. Procédé de préparation de solutions d'acides aminés pour enfants de faible poids de naissance, nouveau-nés à terme et jeunes nourrissons, pouvant être utilisées par voie orale, entérale ou parentérale, procédé caractérisé par le fait que l'on mélange divers acides aminés de manière à obtenir des solutions qui contiennent des acides aminés essentiels (thr, leu, ileu, val, mét, phé, tyr, lys, his) et semi-essentiels, dans un rapport pondéral entre les acides aminés essentiels et semi-essentiels et les acides aminés totaux de 46 à 63 % et de la taurine en une quantité de 0,5 % à 0,7 % en poids par rapport au total des acides aminés.

2. Procédé selon la revendication 1, caractérisé par le fait que les solutions d'acides aminés contiennent de 20,4 à 27,7 % en poids d'acides aminés ramifiés, de 6,0 à 8,1 % en poids d'acides aminés aromatiques, de 2,0 à 2,7 % en poids de méthionine, de 1,6 à 2,2 % en poids de cystéine et de 1,9 à 2,6 % en poids d'ornithine; les pourcentages en poids étant exprimés par rapport au total des acides aminés.

3. Procédé selon la revendication 1 ou 2, caractérisé par le fait qu'on ajoute des solutions d'oses (glucose, maltose, fructose, sorbitol), d'électrolytes, de minéraux, d'oligoéléments, de vitamines et/ou de lipides dans les solutions d'acides aminés.

4. Procédé selon l'une quelconque des revendications 1 à 3, caractérisé par le fait que la concentration en acides aminés est de 2 à 10 g/100 ml de solution.

6

5. Procédé selon l'une quelconque des revendications 1 à 4, caractérisé par le fait qu'on mélange les acides aminés suivants dans les quantités suivantes exprimées en pourcentage en poids:

| | | | |
|---|---|---|---|
| L-Isoleucine | 5,7 | à | 7,7 |
| L-Leucine | 8,5 | à | 11,4 |
| L-Valine | 6,4 | à | 8,7 |
| L-Lysine | 9,3 | à | 12,6 |
| L-Méthionine | 2,0 | à | 2,7 |
| L-Phénylalanine | 3,6 | à | 4,8 |
| L-Thréonine | 3,1 | à | 4,2 |
| L-Tryptophane | 1,7 | à | 2,3 |
| L-Arginine | 7,1 | à | 9,6 |
| L-Histidine | 3,2 | à | 4,4 |
| L-Alanine | 6,8 | à | 9,2 |
| Acide L-aspartique | 5,1 | à | 6,9 |
| L-Cystéine | 1,6 | à | 2,2 |
| Acide L-glutamique | 8,5 | à | 11,4 |
| Glycine | 3,4 | à | 4,6 |
| L-Proline | 2,5 | à | 3,4 |
| L-Sérine | 3,4 | à | 4,6 |
| L-Tyrosine | 0,4 | à | 1,0 |
| L-Ornithine | 1,9 | à | 2,6 |
| Taurine | 0,5 | à | 0,7 |

6. Procédé selon la revendication 1, 2, 3, 4 ou 5, caractérisé par le fait qu'on mélange les acides aminés selon la formule suivante:

| | mg/100 ml |
|---|---|
| L-Isoleucine | 335 |
| L-Leucine | 500 |
| L-Valine | 380 |
| L-Lysine | 550 |
| L-Méthionine | 120 |
| L-Phénylalanine | 210 |
| L-Thréonine | 185 |
| L-Tryptophane | 100 |
| L-Arginine | 420 |
| L-Histidine | 190 |
| L-Alanine | 400 |
| Acide L-aspartique | 300 |
| L-Cystéine (chlorhydrate) | 123 |
| Acide L-glutamique | 500 |
| Glycine | 200 |
| L-Proline | 150 |
| L-Sérine | 200 |
| L-Tyrosine | 45 |
| L-Ornithine | 113 |
| Taurine | 30 |
| Eau q.s. p | 100    ml |

7. Procédé selon la revendication 3, caractérisé par le fait qu'on mélange les composants suivants:

| | |
|---|---|
| solution d'acides aminés de la revendication 6 | 100 ml |
| sodium | 4,4 mEq |
| potassium | 4,7 mEq |
| chlore | 4,4 mEq |
| calcium | 75 mg |
| phosphore | 70 mg |
| magnésium | 7,5 mg |
| zinc | 0,6 mg |
| cuivre | 0,06 mg |

8. Procédé selon la revendication 3, caractérisé par le fait qu'on mélange les composants suivants:

**0 148 680**

| Solution d'acides aminés de la revendication 7 | 50 ml |
|---|---|
| Solution d'ose à 20 % | 100 ml |

9. Procédé selon la revendication 3, caractérisé par le fait qu on mélange les composants suivants:

| Solution d'acides aminés de la revendication 6 | 60 ml |
|---|---|
| Solution de glucose à 30 % | 40 ml |
| Emulsion lipidique à 20 % | 10 ml |

**Patentansprüche** für die Vertragsstaaten: BE, CH, DE, FR, GB, IT, LI, LU, NL, SE

1. Aminosäurelösungen für Kinder mit geringem Geburtsgewicht, ausgetragene Neugeborene und junge Säuglinge, welche Lösungen auf oralem, enteralem oder parenteralem Weg verwendet werden können, dadurch gekennzeichnet, daß sie essentielle Aminosäuren (Thr, Leu, Ileu, Val, Met, Phe, Tyr, Lys, His) und halbessentielle Aminosäuren (Cys, Tau, Tyr) in einem Gewichtsverhältnis von essentiellen und halbessentiellen Aminosäuren zu Gesamtaminosäuren von 46 bis 63 % und Taurin in einer Menge von 0,5 bis 0,7 Gew.-% bezogen auf Gesamtaminosäuren enthalten.

2. Aminosäurelösungen nach Anspruch 1, dadurch gekennzeichnet, daß sie 20,4 bis 27,7 Gew.-% verzweigte Aminosäure, 6,0 bis 8,1 Gew.-% aromatische Aminosäuren, 2,0 bis 2,7 Gew.-% Methionin, 1,6 bis 2,2 Gew.-% Cystein und 1,9 bis 2,6 Gew.-% Ornithin enthalten; wobei die prozentangaben auf Gesamtaminosäuren bezogen sind.

3. Aminosäurelösungen nach Anspruch 1 oder 2, dadurch gekennzeichnet, daß sie Osen (Glucose, Maltose, Fructose, Sorbit), Elektrolyte, Mineralien, Oligoelemente, Vitamine und/oder Lipide enthalten können.

4. Aminosäurelösungen nach irgendeinem der Ansprüche 1 bis 3, dadurch gekennzeichnet, daß die Konzentration der Aminosäuren bei 2 bis 10 g/100 ml Lösung liegt.

5. Aminosäurelösung nach irgendeinem der Ansprüche 1 bis 4, dadurch gekennzeichnet, daß sie die folgenden Aminosäuren in folgenden Mengen, ausgedrückt in Gew.-% bezogen auf Gesamtaminosäuren enthält:

| L-Isoleucin | 5,7 | bis | 7,7 |
|---|---|---|---|
| L-Leucin | 8,5 | bis | 11,4 |
| L-Valin | 6,4 | bis | 8,7 |
| L-Lysin | 9,3 | bis | 12,6 |
| L-Methionin | 2,0 | bis | 2,7 |
| L-Phenylalanin | 3,6 | bis | 4,8 |
| L-Threonin | 3,1 | bis | 4,2 |
| L-Tryptophan | 1,7 | bis | 2,3 |
| L-Arginin | 7,1 | bis | 9,6 |
| L-Histidin | 3,2 | bis | 4,4 |
| L-Alanin | 6,8 | bis | 9,2 |
| L-Asparaginsäure | 5,1 | bis | 6,9 |
| L-Cystein | 1,6 | bis | 2,2 |
| L-Glutaminsäure | 8,5 | bis | 11,4 |
| Glycin | 3,4 | bis | 4,6 |
| L-Prolin | 2,5 | bis | 3,4 |
| L-Serin | 3,4 | bis | 4,6 |
| L-Tyrosin | 0,4 | bis | 1,0 |
| L-Ornithin | 1,9 | bis | 2,6 |
| Taurin | 0,5 | bis | 0,7 |

6. Lösung nach Anspruch 1, 2, 3, 4 oder 5, dadurch gekennzeichnet, daß sie der folgenden Formulierung entspricht:

8

**0 148 680**

|  | mg/100 ml |
|---|---|
| L-Isoleucin | 335 |
| L-Leucin | 500 |
| L-Valin | 380 |
| L-Lysin | 550 |
| L-Methionin | 120 |
| L-Phenylalanin | 210 |
| L-Threonin | 185 |
| L-Tryptophan | 100 |
| L-Arginin | 420 |
| L-Histidin | 190 |
| L-Alanin | 400 |
| L-Asparaginsäure | 300 |
| L-Cystein (Chlorhydrat) | 123 |
| L-Glutaminsäure | 500 |
| Glycin | 200 |
| L-Prolin | 150 |
| L-Serin | 200 |
| L-Tyrosin | 45 |
| L-Ornithin | 113 |
| Taurin | 30 |
| Wasser auf | 100ml |

7. Lösung nach Anspruch 3,
dadurch gekennzeichnet,
daß sie aus folgender Mischung besteht:

| | |
|---|---|
| Aminosäurelösung nach Anspruch 6 | 100 ml |
| Natrium | 4,4 mÄg |
| Kalium | 4,7 mÄg |
| Chlor | 4,4 mÄg |
| Calcium | 75 mg |
| Phosphor | 70 mg |
| Magnesium | 7,5 mg |
| Zink | 0,6 mg |
| Kupfer | 0,06 mg |

8. Lösung nach Anspruch 3,
dadurch gekennzeichnet,
daß sie aus folgender Mischung besteht:

| | |
|---|---|
| Aminosäurelösung nach Anspruch 7 | 50 ml |
| 20 %-ige Ose-Lösung | 100 ml |

9. Lösung nach Anspruch 3,
dadurch gekennzeichnet,
daß sie aus folgender Mischung besteht:

| | |
|---|---|
| Aminosäurelösung nach Anspruch 6 | 60 ml |
| 30 %-ige Glucoselösung | 40 ml |
| 20 %-ige Lipidemulsion | 10 ml |

**Patentansprüche** für den Vertragsstaat: AT

1. Verfahren zur Herstellung von Aminosäurelösungen für Kinder mit geringem Geburtsgewicht, ausgetragene Neugeborene und junge Säuglinge, welche Lösungen auf oralem, enteralem oder parenteralem Weg verabreicht werden können,
dadurch gekennzeichnet,
daß man verschiedene Aminosäuren zur Gewinnung von Lösungen mischt, die essentielle Aminosäuren (Thr, Leu, Ileu, Val, Met, Phe, Tyr, Lys, His) und halbessentielle Aminosäuren in einem Gewichtsverhältnis von essentiellen und halbessentiellen Aminosäuren zu Gesamtaminosäuren von 46 bis 63 % und Taurin in einer Menge von 0,5 bis 0,7 Gew.-%, bezogen auf Gesamtaminosäuren, enthalten.

2. Verfahren nach Anspruch 1,

dadurch gekennzeichnet,
daß die Aminosäurelösungen 20,4 bis 27,7 Gew.-% verzweigte Aminosäuren, 6,0 bis 8,1 Gew.-% aromatische Aminosäuren, 2,0 bis 2,7 Gew.-% Methionin, 1,6 bis 2,2 Gew.-% Cystein und 1,9 bis 2,6 Gew.-% Ornithin enthalten; wobei die Gewichtsprozente auf Gesamtaminosäuren bezogen sind.

3. Verfahren nach Anspruch 1 oder 2,
dadurch gekennzeichnet
daß man Ose-Lösungen (Glucose, Maltose, Fructose, Sorbit), Elektrolyte, Mineralien, Oligoelemente, Vitamine und/oder Lipide zu den Aminosäurelösungen zusetzt.

4. Verfahren nach irgendeinem der Ansprüche 1 bis 3,
dadurch gekennzeichnet,
daß die Konzentration der Aminosäuren 2 bis 10 g/100 ml Lösung beträgt.

5. Verfahren nach irgendeinem der Ansprüche 1 bis 4,
dadurch gekennzeichnet,
daß man die folgenden Aminosäuren in den folgenden, in Gewichtsprozenten ausgedrückten Mengen mischt:

| | | | |
|---|---|---|---|
| L-Isoleucin | 5,7 | bis | 7,7 |
| L-Leucin | 8,5 | bis | 11,4 |
| L-Valin | 6,4 | bis | 8,7 |
| L-Lysin | 9,3 | bis | 12,6 |
| L-Methionin | 2,0 | bis | 2,7 |
| L-Phenylalanin | 3,6 | bis | 4,8 |
| L-Threonin | 3,1 | bis | 4,2 |
| L-Tryptophan | 1,7 | bis | 2,3 |
| L-Arginin | 7,1 | bis | 9,6 |
| L-Histidin | 3,2 | bis | 4,4 |
| L-Alanin | 6,8 | bis | 9,2 |
| L-Asparaginsäure | 5,1 | bis | 6,9 |
| L-Cystein | 1,6 | bis | 2,2 |
| L-Glutaminsäure | 8,5 | bis | 11,4 |
| Glycin | 3,4 | bis | 4,6 |
| L-Prolin | 2,5 | bis | 3,4 |
| L-Serin | 3,4 | bis | 4,6 |
| L-Tyrosin | 0,4 | bis | 1,0 |
| L-Ornithin | 1,9 | bis | 2,6 |
| Taurin | 0,5 | bis | 0,7 |

6. Verfahren nach Anspruch 1, 2, 3, 4 oder 5,
dadurch gekennzeichnet,
daß man die Aminosäuren nach folgender Formulierung mischt:

| | mg/100 ml |
|---|---|
| L-Isoleucin | 335 |
| L-Leucin | 500 |
| L-Valin | 380 |
| L-Lysin | 550 |
| L-Methionin | 120 |
| L-Phenylalanin | 210 |
| L-Threonin | 185 |
| L-Tryptophan | 100 |
| L-Arginin | 420 |
| L-Histidin | 190 |
| L-Alanin | 400 |
| L-Asparaginsäure | 300 |
| L-Cystein (Chlorhydrat) | 123 |
| L-Glutaminsäure | 500 |
| Glycin | 200 |
| L-Prolin | 150 |
| L-Serin | 200 |
| L-Tyrosin | 45 |
| L-Ornithin | 113 |
| Taurin | 30 |
| Wasser auf | 100ml |

7. Verfahren nach Anspruch 3,
dadurch gekennzeichnet,
daß man die folgenden Komponenten mischt:

| | |
|---|---|
| Aminosäurelösung nach Anspruch 6 | 100 ml |
| Natrium | 4,4 mÄg |
| Kalium | 4,7 mÄg |
| Chlor | 4,4 mÄg |
| Calcium | 75 mg |
| Phosphor | 70 mg |
| Magnesium | 7,5 mg |
| Zink | 0,6 mg |
| Kupfer | 0,06 mg |

8. Verfahren nach Anspruch 3,
dadurch gekennzeichnet,
daß man die folgenden Komponenten mischt:

| | |
|---|---|
| Aminosäurelösung nach Anspruch 7 | 50 ml |
| 20 %-ige Ose-Lösung | 100 ml |

9. Verfahren nach Anspruch 3,
dadurch gekennzeichnet,
daß man die folgenden Komponenten mischt:

| | |
|---|---|
| Aminosäurelösung nach Anspruch 6 | 60 ml |
| 30 %-ige Glucoselösung | 40 ml |
| 20 %-ige Lipidemulsion | 10 ml |

**Claims** for the contracting States: BE, CH, DE, FR, GB, IT, LI, LU, NL, SE.

1. Amino acid solutions for children of low birth-weight, neuborn babies at term and young infants, which solutions can be used orally, enterally or parenterally, and are characterized in that they contain essential amino acids, (thr, leu, ileu, val, met, phe, try, lys, his) and semi-essential amino acids (cys, tau, tyr) in a weight ratio between the essential and semi-essential amino acids and the total amino acids of 46 to 63 % and in that they contain taurine in a quantity of 0.5 % to 0.7 % by weight relative to the total amino acids.

2. Amino acid solutions according to claim 1, characterized in that they contain from 20.4 to 27.7 % by weight of branched amino acids, from 6.0 to 8.1 % by weight of aromatic amino acids, from 2,0 to 2,7 % by weight of methionine, from 1,6 to 2,2 % by weight of cysteine, and from 1,9 to 2,6 % by weight of ornithine; percentages by weight being expressed relative to the total amino acids.

3. Amino acid solutions according to claim 1 or 2, characterized in that they can contain monosaccharides (glucose, maltose, fructose, sorbitol), electrolytes, minerals, trace elements, vitamins and/or lipids.

4. Amino acid solutions according to any one of claims 1 to 3, characterized in that the concentration of amino acids is from 2 to 10 g/100 ml of solution.

5. Amino acid solution according to any one of claims 1 to 4 characterized in that it contains the following amino acids im the following amounts, expressed as percentages by weight relative to the total amino acids:

| L-Isoleucine | 5,7 | to | 77 |
|---|---|---|---|
| L-Leucine | 8,5 | to | 11,4 |
| L-Valine | 6,4 | to | 8,7 |
| L-Lysine | 9,3 | to | 12,6 |
| L-Methionine | 2,0 | to | 2,7 |
| L-Phenylalanine | 3,6 | to | 4,8 |
| L-Threonine | 3,1 | to | 4,2 |
| L-Tryptophane | 1,7 | to | 2,3 |
| L-Arginine | 7,1 | to | 9,6 |
| L-Histidine | 3,2 | to | 4,4 |
| L-Alanine | 6,8 | to | 9,2 |
| L-Aspartic acid | 5,1 | to | 6,9 |
| L-Cysteine | 1,6 | to | 2,2 |
| L-Glutamic acid | 8,5 | to | 11,4 |
| Glycine | 3,4 | to | 4,6 |
| L-Proline | 2,5 | to | 3,4 |
| L-Serine | 3,4 | to | 4,6 |
| L-Tyrosine | 0,4 | to | 1,0 |
| L-Ornithine | 1,9 | to | 2,6 |
| Taurine | 0,5 | to | 0,7 |

6. Solution according to claim 1, 2, 3, 4 or 5, characterized in that it has the folloving formula:

| | mg/100 ml |
|---|---|
| L-Isoleucine | 335 |
| L-Leucine | 500 |
| L-Valine | 380 |
| L-Lysine | 550 |
| L-Methionine | 120 |
| L-Phenylalanine | 210 |
| L-Threonine | 185 |
| L-Tryptophane | 100 |
| L-Arginine | 420 |
| L-Histidine | 190 |
| L-Alanine | 400 |
| L-Aspartic acid | 300 |
| L-Cysteine (hydrochloride) | 123 |
| L-Glutamic acid | 500 |
| Glycine | 200 |
| L-Proline | 150 |
| L-Serine | 200 |
| L-Tyrosine | 45 |
| L-Ornithine | 113 |
| Taurine | 30 |
| Water q.s. | 100ml |

7. Solution according to Claim 3, characterized in that it consists of the following mixture:

| Amino acid solution of Claim 6 | 100 ml |
|---|---|
| Sodium | 4,4 mEq |
| Potassium | 4,7 mEq |
| Chlorine | 4,4 mEq |
| Calcium | 75 mg |
| Phosphorus | 70 mg |
| Magnesium | 7.5 mg |
| Zinc | 0,6 mg |
| Copper | 0,06 mg |

8. Solution according to Claim 3, characterized in that it consists of the following mixture:

| Amino acid solution of Claim 7 | 50 ml |
|---|---|
| 20 % strength monosaccharide solution | 100 ml |

9. Solution according to Claim 3, characterized in that it consists of the following mixture:

| | |
|---|---|
| Amino acid solution of Claim 6 | 60 ml |
| 30 % strength glucose solution | 40 ml |
| 20 % strength lipid emulsion | 10 ml |

**Claims** for the contracting State: AT

1. Process for the preparation of amino acid solutions for children of low birth-weight, newborn babies at term and young infants, which solutions can be used orally, enterally or parenterally, and is characterized in that several amino acids are mixed in such a way as to obtain solutions which contain essential amino acids (thr, leu, ileu, val, met, phe, tyr, lys, his) and semi-essential amino acids, in a weight ratio between the essential and semi-essential amino acids and the total amino acids of 46 to 63 % and of taurine in a quantity of 0.5 % to 0.7 % by weight relative to the total of the amino acids.

2. Process according to Claim 1, characterized in that the amino acid solutions contain from 20,4 to 27,7 % by weight of branched amino acids, from 6,0 to 8,1 % by weight of aromatic amino acids, from 2,0 to 2,7 % by weight of methionine, from 1,6 to 2,2 % by weight of cysteine and from 1,9 to 2,6 % by weight of ornithine; percentages by weight being expressed relative to the total amino acids.

3. Process according to Claim 1 or 2, characterized in that solutions of monosaccharides (glucose, maltose, fructose, sorbitol), of electrolytes, of minerals, of trace elements, of vitamins and/or of lipids are added to the amino acid solutions.

4. Process according to any one of Claims 1 to 3, characterized in that the concentration of amino acids is from 2 to 10 g/100 ml of solution.

5. Process according to any one of claims 1 to 4, characterized in that the following amino acids are mixed in the following amounts, expressed as percentages by weight:

| | | | |
|---|---|---|---|
| L-Isoleucine | 5,7 | to | 7,7 |
| L-Leucine | 8,5 | to | 11,4 |
| L-Valine | 6,4 | to | 8,7 |
| L-Lysine | 9,3 | to | 12,6 |
| L-Methionine | 2,0 | to | 2,7 |
| L-Phenylalanine | 3,6 | to | 4,8 |
| L-Thronine | 3,1 | to | 4,2 |
| L-Tryptophane | 1,7 | to | 2,3 |
| L-Arginine | 7,1 | to | 9,6 |
| L-Histidine | 3,2 | to | 4,4 |
| L-Alanine | 6,8 | to | 9,2 |
| L-Aspartic acid | 5,1 | to | 6,9 |
| L-Cysteine | 1,6 | to | 2,2 |
| L-Glutamic acid | 8,5 | to | 11,4 |
| Glycine | 3,4 | to | 4,6 |
| L-Proline | 2,5 | to | 3,4 |
| L-Serine | 3,4 | to | 4,6 |
| L-Tyrosine | 0,4 | to | 1,0 |
| L-Ornithine | 1,9 | to | 2,6 |
| Taurine | 0,5 | to | 0,7 |

6. Process according to Claim 1, 2, 3, 4 or 5, characterized in that the amino acids are mixed according to the following formula:

|  | mg/100 ml |
| --- | --- |
| L-Isoleucine | 335 |
| L-Leucine | 500 |
| L-Valine | 380 |
| L-Lysine | 550 |
| L-Methionine | 120 |
| L-Phenylalanine | 210 |
| L-Threonine | 185 |
| L-Tryptophane | 100 |
| L-Arginine | 420 |
| L-Histidine | 190 |
| L-Alanine | 400 |
| L-Aspartic acid | 300 |
| L-Cysteine (hydrochloride) | 123 |
| L-Glutamic acid | 500 |
| Glycine | 200 |
| L-Proline | 150 |
| L-Serine | 200 |
| L-Tyrosine | 45 |
| L-Ornithine | 113 |
| Taurine | 30 |
| Water q.s. | 100ml |

7. Process according to Claim 3, characterized in that the following constituents are mixed:

| Amino acid solution of Claim 6 | 100 ml |
| --- | --- |
| Sodium | 4,4 mEq |
| Potassium | 4,7 mEq |
| Chlorine | 4,4 mEq |
| Calcium | 75 mg |
| Phosphorus | 70 mg |
| Magnesium | 7,5 mg |
| Zinc | 0,6 mg |
| Copper | 0.06 mg |

8. Process according to Claim 3, characterized in that the following constituents are mixed:

| Amino acid solution of Claim 7 | 50 ml |
| --- | --- |
| 20 % strength monosaccharide solution | 100 ml |

9. Process according to Claim 3, characterized in that the following constituents are mixed:

| Amino acid solution of Claim 6 | 60 ml |
| --- | --- |
| 30 % strength glucose solution | 40 ml |
| 20 % strength lipid emulsion | 10 ml |